(19) Europäisches Patentamt
European Patent Office
Office européen des brevets

(11) Publication number: 0 409 473 A2

# (12) EUROPEAN PATENT APPLICATION

(21) Application number: 90307575.2

(22) Date of filing: 11.07.90

(51) Int. Cl.5: C07H 15/10, A61K 31/70, A61K 31/72

(30) Priority: 12.07.89 AR 314371

(43) Date of publication of application: 23.01.91 Bulletin 91/04

(84) Designated Contracting States: AT BE CH DE DK ES FR GB GR IT LI LU NL SE

(71) Applicant: Mammarella, Carlos
San Juan 2266
Buenos Aires(AR)

Applicant: Rodriguez, Pablo
San Juan 2266
Buenos Aires(AR)

Applicant: Cumar, Federico A.
P.O. Box 61
Cordoba, Cordoba(AR)

(72) Inventor: Mammarella, Carlos
San Juan 2266
Buenos Aires(AR)
Inventor: Rodriguez, Pablo
San Juan 2266
Buenos Aires(AR)
Inventor: Cumar, Federico A.
P.O. Box 61
Cordoba, Cordoba(AR)

(74) Representative: Pearce, Anthony Richmond et al
MARKS & CLERK Alpha Tower Suffolk Streeteet
Queensway Birmingham B1 1TT(GB)

## (54) A process for obtaining the gangliosides from nervous tissue of mammalians and for the treatment of psychoorganic diseases with said material compositons.

(57) A process for obtaining gangliosides from mammalian nervous tissue, comprises the step of:-

(a) extracting the nervous tissue using at least one ($C_1$-$C_4$) alcohol and at least one non-polar organic solvent,

(b) filtering the mixture and adding water to the filtrate to produce an aqueous alcoholic phase,

(c) adsorbing gangliosides present in said aqueous alcoholic phase on an ion exchange material, and then washing the ion exchange material with a ketone,

(d) recovering the gangliosides from the ion exchange material using a solvent mixture including water, ($C_1$-$C_4$) alcohol(s), organic solvent(s) and alkali(es),

(e) purifying the material recovered from the ion exchange material by partition or absorption column chromatography, and

(f) recovering the extracted gangliosides from the chromatography column.

EP 0 409 473 A2

# A PROCESS FOR OBTAINING THE GANGLIOSIDES FROM NERVOUS TISSUE OF MAMMALIANS AND FOR THE TREATMENT OF PSYCHOORGANIC DISEASES WITH SAID MATERIAL COMPOSITIONS

This invention is concerned with a process for obtaining mixtures of gangliosides from mammalian nervous tissue and for their use in the treatment of psycho-organic diseases.

Furthermore, this invention provides compositions which include fractions or mixtures of gangliosides extracted from mammalian nervous tissue, having pharmacological activity and being useful as adjuvants for the treatment of diseases of the mood which require the use of monoamine oxidase inhibitors or neuronal uptake blockers of noradrenaline or serotonin. Likewise, they are useful for treating psycho-organic diseases which require the recovery of damaged neurons and stimulation of neuronal plasticity.

## BACKGROUND OF THE INVENTION

It is known that gangliosides comprise one of the three groups constituting the sphingolipids family, complex lipids which are present in great amounts in the nervous tissues (brain, cerebral cortex, spinal cord, etc.), and only in small amounts in the adipose tissue.

The sphingolipids contain three building blocks: a fatty acid molecule ($C_{18}$-$C_{20}$) linked by means of peptide bond to an aminoalcohol, generally named "sphingosine" (of which no less than 30 different species are known), making up units called "ceramides", for example:-

$$CH_3(CH_2)_{12}-CH=CH-\underset{OH}{\overset{3}{CH}} - \underset{NH-CO-(CH_2)_{16}-CH_3}{\overset{2}{CH}}-\overset{1}{CH_2}-OH$$

is a ceramide of stearic acid and sphingosine.

The third building block arises from substitution of the hydroxy group at the $C_1$ position.

The esterification of the 1-alcohol group with phosphorylcholine or phosphorylethanolamine leads to the formation of an important group of lipids called sphingomyelins, present in high proportion in mammalian nervous tissue. Example:

$$-O-\underset{\underset{O}{\|}}{\overset{O^-}{P}}-O-(CH_2)_2-\overset{+}{N}(CH_3)_3 \qquad \text{(phosphorylcholine)}$$

Another group of complex lipids of this type, are the "neutral glycosphingolipids", wherein the alcoholic function at the $C_1$ position is blocked by a glucoside bond to a neutral carbohydrate residue (galactose, glucose, etc.). These compounds are present in high quantities in neural tissue and to a lesser extent in non-neural tissue.

A third group of compounds included under the donomination of sphingolipids, are the "acid glycosphingolipids" usually called "gangliosides", wherein the 1-alcohol function is also blocked by a glucoside bond with an oligosaccharide molecule carrying sialic acid. Of these acids, N-acetyl neuraminic acid is usually found in human gangliosides, N-glycolyl neuraminic acid also being quite common.

More than 20 different types of gangliosides have been identified, which differ in the quantity and in the relative positions of the hexose and the sialic acid residues.

Gangliosides containing only one fragment of sialic acid are called monosialogangliosides; if they contain 2, they are called disialogangliosides; if they contain 3, they are called trisialogangliosides, and if they contain 4, they are called tetrasialogangliosides. They are indicated as GM, GD, GT and GQ, respectively. A numerical suffix and a letter are added. The numerical suffix is equal to 5-n, wherein n is the number of neutral hexose units. The letters (generally a, b) indicate isomeric positions of the sialic acids fragments. These fragments have different Rf values in thin layer chromatography.

Examples:

GM:$_1$ monosialoganglioside with four units of neutral hexose (n = 4), see the TABLE hereinafter.

$GD_{1a}$: disialoganglioside, wherein n = 4, and has specific distributions of sialic acid fragments.

$GD_{1b}$: disialoganglioside, wherein n = 4 and exhibits isomerism in connection to the position of the sialic acid fragment, with respect to $GD_{1a}$.

The importance of gangliosides in the transmission of the nerve impulse through the synapsis and the immunity reactions of tissue and cell-to-cell recognition is known.

Moreover, taking into account the high proportion of gangliosides in relation to other sphingolipids in the membranes of the neurons, it has been established that they accomplish an important function in the structure and function thereof. Exogenously added, gangliosides are effective for inducing functional changes in synaptosomes and plastic changes in neurons cultured in vitro , existing numerous works which demonstrate this action [ B. Maggio, F.A. Cumar, R. Caputto, Biochem-Biophys Acta 1981, 650, 69-87, and M.C. Byrne, R.W. Ledeen, F.J. Raisen, G. Yorkee and J.R. Scalafani, J. Neurochem. 1983 41 1214-1222 ]. These effects, which may be observed in a tissue culture, are also verifiable when they are injected in vivo into the animal under experimentation. Thus, several groups of researchers in different parts of the world have demonstrated that exogenous administration of gangliosides facilitates the repair of neuronal tissue which has been injured by different types of noxa [ H.A. Tilson, G.J. Harry, K. Nanry, P.M. Hudson, J.S. Hong, J of Neurosc. Res. 1988, 19 , 88-93 ]. However, all works above mentioned limit the field of action of gangliosides to anatomical repair of the injured cell and, therefore, restrict the potential medical use thereof to structural alterations of neurons, cause by trauma, metabolic or toxic effect such as different peripheral neuropathies, caused by diabetes, alcoholism, anti-neoplastic drugs, trauma, etc, and therefore can be said to restrict the therapeutic field of action of gangliosides [ R. Abraham, D.M. Levy and R.M. Abraham, Diabetes Research 1988, 7 , 129-135 ].

It has now been proved that it is possible to apply gangliosides to facilitate functional neuronal plasticity. Basic experimental evidence shows that gangliosides facilitate a series of behavioural responses connected with neuronal plasticity [ H. Rahmann, in Learning and memory: mechanisms of information storage in the nervous system, ed. H. Matthies Pergamon Press, N. Y. 1986, p.235-245 ].

The proposal that the mixture of gangliosides is an experimental medicine which has been developed for the treatment of afflictions related to behaviour is an hypothesis confirmed by definite experimental evidence.

As regards the extraction methods of gangliosides known up to the moment, they use numerous steps for obtaining a crude product, including the employment of both peroxide-forming solvents as well as different types of ethers. The peroxides can react with the double bonds reducing the yield of the final products.

The purification known until now consists basically in precipitation or dialysis. Precipitation always leaves some active product in solution and also drags out impurities, thus increasing the number of purification steps needed. Dialysis is long and expensive and also leads to losses in the yield of active product.

## DISCLOSURE OF THE INVENTION

An object of the present invention is to provide an improved process for obtaining gangliosides from mammalian nervous tissue for the treatment of psycho-organic diseases, which require neuronal plasticity recovery or the use of monoamine-oxidase inhibitors or neuronal uptake blockers of noradrenaline or serotonine.

Thus, in one aspect, the present invention resides in a process for obtaining gangliosides from mammalian nervous tissue, comprising the steps of:-

(a) extracting the nervous tissue using at least one ($C_1$-$C_4$) alcohol and at least one non-polar organic solvent,

(b) filtering the mixture and adding water to the filtrate to produce an aqueous alcoholic phase,

(c) adsorbing gangliosides present in said aqueous alcoholic phase on an ion exchange material (preferably an anionic exchange material), and then washing the ion exchange material with a ketone,

(d) recovering the gangliosides from the ion exchange material using a solvent mixture including water, ($C_1$-$C_4$) alcohol(s), organic solvent(s) and alkali(es),

(e) purifying the material recovered from the ion exchange material by partition or absorption column chromatography, and

(f) recovering the extracted gangliosides from the chromatography column.

Preferably, the gangliosides are recovered in step (d) from the ion exchange material by eluting them with said solvent mixture, distilling the solvent mixture, and extracting the residue with a solvent mixture including water, ($C_1$-$C_4$) alcohol(s) and non-polar organic solvent(s), the solvent mixture extract being subjected to the partition or absorption column chromotography of purifying step (e).

The alkali is preferably a volatile alkali such as ammonia or an amine.

The recovering step (h) is preferably effected by eluting the column with a mixture of water, ($C_1$-$C_4$) alcohol(s) and non-polar organic solvent(s) using solvent mixtures of increasing polarity, and recovering the fractions which contain the gangliosides.

Preferably, the fractions which contain gangliosides are subjected to evaporation to evaporate the solvent mixture, and the residue that contains the extracted gangliosides is recovered.

The ion exchange material of step (b) is preferably a dextran- or cellulose-based material (eg DEAE Sephadex or DEAE cellulose, dimethylaminoethycellulose) preferably applied at a ratio of 0.8-1.5 g for each 0.1 g of.gangliosides to be adsorbed.

Another object of the present invention is to obtain an active substance composition having pharmacological activity useful as an adjuvant in the treatment of disorders of the mood ascribed to the block of neuronal uptake noradrenaline or serotonine or in diseases treated with monoamine-oxidase inhibitors, and even useful for treating psychosomatic diseases which require the re-establishment of neuronal plasticity.

Thus, in another aspect of the present invention, the present invention provides a composition having pharmacological activity useful as an adjuvant in the treatment of mood disorders such as unipolar endogenous depression, bipolar endogenous depression, melancholia or reactive depression treated with neuronal uptake blockers of noradrenaline or serotonine or in diseases treated with monoamino-oxidase inhibitors, or the treatment of psychosomatic diseases which require the recovery of neuronal, said composition comprising, in an administrable form, gangliosides extracted from mammalian nervous tissue in a pharmaceutically acceptable vehicle.

## DETAILED DESCRIPTION OF THE INVENTION

The method for obtaining gangliosides according to the present invention, avoids the previously mentioned disadvantageous prior art steps, and proposes an extraction and partition between a non-polar organic solvent phase and an aqueous alcoholic phase [ J. Folch, M. Lees and G.B. Sloane-Stanley, J. Biol. Chem., 226 , 497 (195)] followed by a purification in an ion exchange modified cellulose (DEAE Sephadex), and a selective elution thereof, under such conditions that the yield is higher than the previously described methods and the costs are lower.

The ratio of gangliosides to ion exchange material (eg DEAE Sephadex) can be significantly higher than those described by Takashi Momol, Susumo Ando and Yoshitaka Nagai in Biochim. Biophys., Acta 441 448/97 (1976). The solvents for elution proposed do not contain salts, thus avoiding desalination steps (dialysis). Therefore, our production method results in a process which is simpler than those used until now, simplifying some stages and eliminating the more complicated ones. These stages can be performed in a bathwise manner, avoiding the use of columns which demand more time. Moreover, and concerning the economy of the process, the exchange material used (preferably DEAE Sephadex) is left in such a condition that it can be regenerated by washing it with dilute acetic acid solution, afterwards with water, and finally with methanol-water mixture and then reused. By washing the DEAE Sephadex that retains the gangliosides, with solvents having a carbonyl group, a substantial proportion of coloured material dragged out as an impurity is eliminated.

The eluted product is finally purified by chromatography on silica gel or any other suitable solid support, eg a natural or synthetic silicate such as HYFLOW or CELITE. Due to its nature, our method allows a yield which is higher than the methods described by Lars Svennerholm in J. Neurochem. 10 , 613 (1963) or in Belgium Patent (Fidia) 843695 and Patent 7540170, thereby ensuring that the final product will have a higher quality.

In the first stage, that is to say, extraction, aromatic hydrocarbons, such as benzene or toluene, or aliphatic hydrocarbons, such as n-heptane or n-hexane, or mixtures of isomers of hexanes, or chlorinated solvents such as dichloromethane, chloroform, 1,1-dichloroethane 1,2-dichloroethane, dichlorethylene, trichloroethane, 2-chloropropane, 1-chloropropane, etc. can be used as non-polar organic solvents. The alcohols used to form the aqueous alcoholic phase can be monohydric alcohols ( eg $C_1$ -$C_4$ alcohols such as methanol, ethanol, n-propanol, 2-propanol, n-butanol, isobutanol (2-butanol), methyl cellosolve or ethylcellosolve ) or dihydric alcohols ( eg $C_2$ -$C_3$ alcohols such as ethyleneglycol, 1,3-dihydroxypropane, or 1,2-dihydroxypropane ).

The following examples are given solely for the purpose of illustrating the different ways that exist to carry out the present invention.

EXAMPLE 1

100 g of homogenized bovine encephalic matter are placed in a 2 liter beaker, and 196 ml of isopropanol are added with stirring to produce a homogenous paste to which 265 ml of toluene are added with continued stirring. The beaker is covered and stirring is continued for 2 hours. Then, it is filtered under vacuum.

To the clear liquid derived from the previous filtration, water is added at the ratio of about 22 ml for each 100 ml of filtrate. After smooth stirring to secure mixing, it is left standing for 24 hours. The phases are separated and the organic solvent phase is discarded, leaving the aqueous alcoholic phase available for subsequent operations.

EXAMPLE 2

Example 1 is repeated, but using 265 ml of dichloromethane as the non-polar organic solvent, and 200 ml of methylcellosolve (ethyleneglycol monomethylether) as the alcohol.

EXAMPLE 3

Example 1 is repeated, but using 265 ml of 1,2-dichlorethane as the non-polar organic solvent, and 130 ml of n-butanol as the alcohol.

EXAMPLE 4

Example 1 is repeated, but using 320 ml of chloroform as the non-polar organic solvent, and 260 ml of methanol as the alcohol.

EXAMPLE 5

Example 1 is repeated, but using dichloroethylene as the inert solvent, and isobutanol as the alcohol.

EXAMPLE 6

The aqueous alcoholic phase obtained according to Example 1, 2, 3, 4 or 5 is treated with DEAE Sephadex at the ratio of 0.8 g for each 120 mg of gangliosides to be absorbed in a 1-litre beaker, for 20 hours under mild stirring. Then, it is vacuum filtered using a Buchner-type funnel, discarding the filtrate. Afterwards, the DEAE Sephadex is washed over the filter with a solvent containing a carbonyl group (acetone).

Gangliosides are eluted from the DEAE Sephadex by washing the latter with a mixture (56: 35: 3: 2 by vol.) of dichloroethane: isopropanol: water: methylamine (40%), in a batchwise manner for 20 minutes, the water: methylamine (40%) corresponding to a 40%w/w solution of methylamine in water. The liquors are gathered and filtered.

EXAMPLE 7

In a modification Example 6, the DEAE Sephadex is washed with a mixture (60: 36: 0.85: 2.2 by vol.) of methylene chloride: n-butanol: water: methylamine 40%.

EXAMPE 8

In a further modification of Example 6, the DEAE Sephadex is washed with a mixture (58.3: 33.8: 4.17: 3.6 by vol.) of chloroform: methanol: water: ammonia, the water-ammonia system corresponding to a 25%w/w solution of ammonia in water.

EXAMPLE 9

The elution solvent obtained according to Example 6, 7 or 8 is removed from the extracted gangliosides by vacuum evaporation at 40 °C in a rotary evaporation until dryness is achieved. The residue is redissolved in a mixture (74.5: 23: 2.5 by vol.) of chloroform: methanol: distilled water.

Afterwards, a silica gel column is prepared using the solvent described in the previous step.

The solvent is drained off up to dryness. The residue is taken up in a small amount of the same solvent and the obtained ganglioside solution is seeded in the column. It is then eluted with an additional amount of the same solvent, continuing with increasing polar mixtures of chloroform: methanol: distilled water.

10 ml fractions are collected, and spectrophotometrically screened for phospholipids and gangliosides according to Lars Svennarholm in Biochim. Biophys. Acta 24 , 604 (1957) using a color reaction. Constant flow is maintained in the column. The fractions richer in gangliosides are selected and then gathered. The gathered fractions are filtered and evaporated to dryness under vacuum in a rotary evaporator. They are taken up again in distilled water, and the pH is adjusted to 7.8 using diluted solutions of hydrochloric acid and sodium hydroxide and then are lyophilized. These operations yield between 90 and 180 mg of gangliosides per 100 g fresh tissue.

Usual experimental yield: 150-170 mg.

This yield is at least three times higher than the yield achieved in Belgian Patent No. 843695.

BIOLOGICAL EFFECTS

Today the term "neuronal plasticity" indicates the ability of the nervous system to change or modify its accquired responses to enviromental stimuli, exogenous or endogenous stimulations, or traumatic and/or toxic injuries. In short, the plasticity of the central nervous system is the condition thereof to permit the repair of damage tissue, give organic base for the memory process and respond to therapeutics with behavioural changes.

The clearest example of plastic or adaptive reactions in response to a pharmacological treatment and connected with a therapeutic effect is undoubtedly the one which takes place during the treatment with antidepressant drugs.

Disorders of mood or behaviour comprise the major endogenous depression and the bipolar depression or maniac-depressive diseases which are mainly characterized by changes in character, comprising feelings of deep sadness, mental slowness and loss of concentration, pessimistic worries and an intense sense of minus value. These physical changes are accompanied by various organic signs, such as a characteristic insomnia, anorexia, asthenia, loss of weight and libido diminution and interruption of the hormonal circadian cycles. All these signs and symptoms are accompanied by suicidal tendencies, suicide being a certain and relatively high risk probability (15%).

Many of the drugs used to treat endogeneous depression exert deep effects on the metabolism of a group of chemical substances, that is, the monoamines: noradrenaline and serotonine, which act as neurotransmitters in the Central Nervous System. The more conspicuous effect is to block the neuronal uptake mechanisms for amines. When the neuronal uptake is blocked or impaired, the effect of these neurotransmitters on postsynaptic or effector neurons is enhanced, being both potentiated in magnitude and prolonged in time.

Over the period 1960-1975, the pharmacodynamic basis for the therapeutic action of antidepressants was ascribed to this blocking of uptake. However, various inconsistencies demonstrated that the phenomenon was more complex. Thus, for example, although the blocking of the monoamine inactivation is immediate upon the administration of the drug, the clinical therapeutic response is only achieved after a period of about 15 days of continuous treatment. Furthermore, several drugs that also cause the inhibition of the uptake, such as amphetamines or cocaine are not antidepressant and, even though they produce behavioural activation when administered in an acute manner, they lack clinical efficacy as antidepressants. On the contrary, the antidepressants produce sedation and somnolence at the beginning of the treatment.

Later studies allow a more complex theory on the mechanism of action of the antidepressants to be conceived. To a primary effect related to the increase in the monoaminergic neurotransmission, there follows an adaptive or plastic response of certain neuronal circuits which involve a decrease in the number of receptors betaadrenergic and serotonergic. These plastic responses, which take more than two weeks to be completed, would be the organic substrate to the therapeutic response to antidepressants.

On the basis of these new theories, the methods for experimental searches of antidepressants have varied. Formerly, the acute affect connected in principle with the evaluation of the effectiveness as blockers of the inactivation mechanism was investigated. At present, instead, there is an attempt to evaluate the effect of an extended treatment of the animal over certain behaviours, thus seeking the results of plastic changes which took place following the treatment.

The forced swimming test is an example of this methodology. The animal under experimentation is placed in a container filled with water under controlled temperature, where it can swim but lacks any possibility to escape. After several minutes of forced swimming, the animal accepts the impossibility of escaping and ceases its efforts to escape, letting itself float. When this test is repeated 10 days later, the animal does not try to swim, thus accepting the lack of escape, with its immobility. If in the interval between both tests, the animal has received treatment with an antidepressant, the animal swims vigorously, trying to find an outlet. This test is very sensitive and allows the gradual obtention of responses (time of swimming), which are proportional to the administered doses (dose-response curve) or to the duration of treatment (time-response curve) [ R.D. Porsolt, G. Anton, N. Blavet and M. Jalfre, European J. Pharmacol., 1978, 47 , 379-383 ].

The administration of gangliosides together with antidepressant drugs to animals under experimentation, reduces the dose of antidepressant and time of treatment required to obtain the intended effect. This potentiation would be related to the facilitation of the plastic phenomena of the central nervous system produced by gangliosides [ V.A. Molina, E.A. Keller and O.A. Orsingher, European J. Pharmacol., 1989, 160 , 247-252).

These and other similar experiments lead to the conclusion that the administration of a ganglioside mixture can improve the depressed patient's therapeutic response to the known antidepressant. It is proposed then:

1. The use of a ganglioside mixture (extracted, for example, from bovine, porcine, equine, ovine encephalon), containing mainly GM1, GD1a, GD1b, and GT1b to facilitate the therapeutic effect of antidepressant drugs, such as dimethylimipramine, imipramine, nortiptiline, maproptiline, pargyline, etc.

2. The above mentioned use may be applicable to reduce the dosage of antidepressant or reduce the time necessary between the beginning of the treatment and the therapeutic effect of the antidepressant.

3. The concomitant use of gangliosides with other pharmacological therapeutics, causing adaptive effects on the central nervous system, such as:
anti-epilectic drugs: diphenylhydantione, carbamezepine,
nootropic drugs:piracetam
antipsychotic drugs: chloropromazine, etc.

4. The use of the ganglioside mixture in the treatment of drug dependence syndromes to facilitate the adaptive response, during the period of drug withdrawal such as: morphine, heroin, marihuana, cocaine, amphetamines and diethylamine derivates of lysergic acid, etc.

Before using said ganglioside mixture, the following variables were studied: Acute, subacute and chronic toxicity in mice and rats and presence of pyrogen or histamine-like substances. These last two assays were "negative", indicating the purity of the mixture. As regards toxicity, the lethal dose 50% ($LD_{50}$) obtained was about 2.5 g/kg, indicating a wide therapeutic index.

TABLE

THE STRUCTURE OF GM1 IS SHOWN BELOW AS A TYPICAL EXAMPLE OF GANGLIOSIDES

←hydrophilic oligosaccharide fragment →|← hydrophobic ceramide fragment→

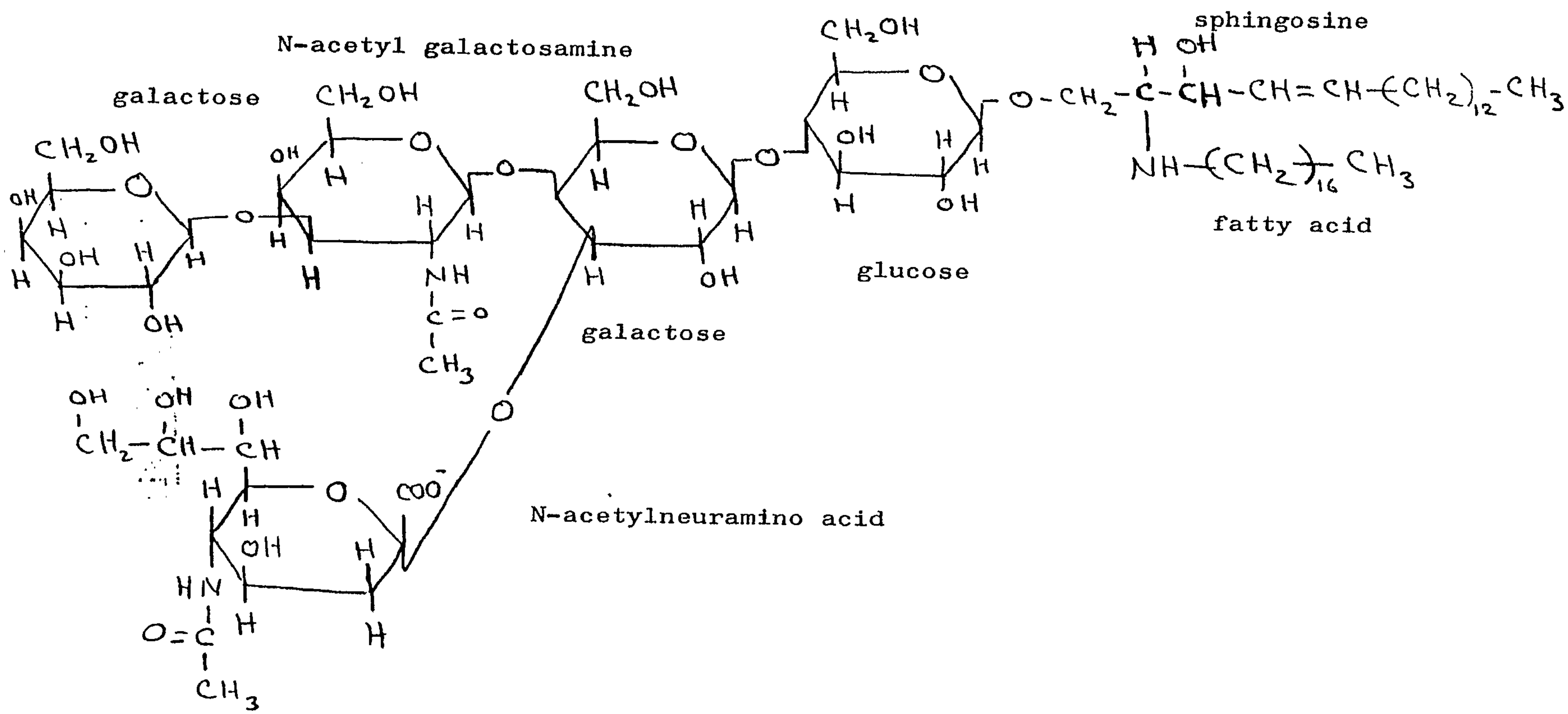

sphingosine

fatty acid

N-acetyl galactosamine

galactose

galactose

glucose

N-acetylneuramino acid

## Claims

1. A process for obtaining gangliosides from mammalian nervous tissue, comprising the steps of:-
(a) extracting the nervous tissue using at least one ($C_1$-$C_4$) alcohol and at least one non-polar organic solvent,
(b) filtering the mixture and adding water to the filtrate to produce an aqueous alcoholic phase,
(c) adsorbing gangliosides present in said aqueous alcoholic phase on an ion exchange material, and then washing the ion exchange material with a ketone,
(d) recovering the gangliosides from the ion exchange material using a solvent mixture including water, ($C_1$-$C_4$) alcohol(s), organic solvent(s) and alkali(es),
(e) purifying the material recovered from the ion exchange material by partition or absorption column chromatography, and
(f) recovering the extracted gangliosides from the chromatography column.

2. A process as claimed in claim 1, wherein the gangliosides are recovered in step (d) from the ion exchange material by eluting them with said solvent mixture, distilling the solvent mixture, and extracting the residue with a solvent mixture including water, ($C_1$-$C_4$) alcohol(s) and non-polar organic solvent(s), the solvent mixture extract being subjected to the partition or absorption column chromotography of purifying step (e).

3. A process according to claim 2, wherein the alkali is a volatile alkali.

4. A process as claimed in claim 1, 2 or 3, wherein the recovering step (h) is effected by eluting the column with a mixture of water, ($C_1$-$C_4$) alcohol(s) and non-polar organic solvent(s) using solvent mixtures of increasing polarity, and recovering the fractions which contain the gangliosides.

5. A process as claimed in claim 4, wherein the fractions which contain gangliosides are subjected to evaporation to evaporate the solvent mixture, and the residue that contains the extracted gangliosides is recovered.

6. A process as claimed in claim 5, wherein the recovered residue is redissolved in an aqueous medium and then lyophilized.

7. A process according to any preceding claim, wherein the nervous tissue is bovine encephalic matter, spinal cord or cerebellum.

8. A process according to any preceding claim wherein, in the extracting step (a) the ($C_1$-$C_4$) alcohol is isopropanol, and the non-polar organic solvent is toluene, dichloromethane, chloroform, 1,2-dichloroethane or dichloroethylene.

9. A process according to any one of claims 1 to 7, wherein in the extracting step (a), ethyleneglycol monomethylether (methylcellosolve) is used as the alcohol.

10. A process according to any one of claims 1 to 7, wherein in the extracting step (a), methanol is used as the alcohol and chloroform is used as the non-polar organic solvent.

11. A process according to any preceeding claim, wherein the ion exchange material of step (b) is a dextran- or cellulose-based material applied at a ratio of 0.8-1.5 g for each 0.1 g of gangliosides to be adsorbed.

12. A process according to any preceding claim, wherein the ketone is acetone.

13. A process according to any preceding claim, wherein the solvent mixture used to elutes the ionic exchange material is a mixture of water, at least one ($C_1$-$C_4$) alcohol, methylamine and/or ammonia, and dichloroethane, methylene chloride or chloroform.

14. A process according to claim 2 or 5, wherein the evaporation of the solvent mixture is achieved by means of vacuum evaporation to dryness of the obtained solution at 30° C - 45° C.

15. A process as claimed in claim 2, wherein the residue obtained in step (d) is redissolved in a solvent mixture of chloroform: methanol: water.

16. The use of gangliosides obtained by a process as claimed in any preceding claim for the manufacture of a medicament for the treatment of psycho-organic diseases, which require the recovery of neuronal plasticity or the use of monoamino-oxidase inhibitors or neuronal receptor deblockers of noradrenaline or serotonine.

17. A composition having pharmacological activity useful as an adjuvant in the treatment of mood disorders such as unipolar endogenous depression, bipolar endogenous depression, melancholia or reactive depression treated with neuronal uptake blockers of noradrenaline or serotonine or in diseases treated with monoamino-oxidase inhibitors, or the treatment of psychosomatic diseases which require the recovery of

neuronal, said composition comprising, in an administrable form, gangliosides extracted from mammalian nervous tissue in a pharmaceutically acceptable vehicle.

18. A copmposition according to claim 17, wherein said nervous tissue is the encephalic mass, the spinal cord and/or the cerebellum of a bovine animal.